(19) Europäisches Patentamt
European Patent Office
Office européen des brevets

(11) Publication number: 0 107 875 B1

(12) EUROPEAN PATENT SPECIFICATION

(45) Date of publication of patent specification: 04.04.90

(51) Int. Cl.$^5$: C 07 C 2/76, B 01 J 29/04

(21) Application number: 83201420.3

(22) Date of filing: 04.10.83

# (54) Process for the preparation of an aromatic hydrocarbon mixture.

(30) Priority: 28.10.82 NL 8204166
30.11.82 NL 8204633

(43) Date of publication of application:
09.05.84 Bulletin 84/19

(45) Publication of the grant of the patent:
04.04.90 Bulletin 90/14

(84) Designated Contracting States:
BE DE FR GB IT NL

(56) References cited:
EP-A-0 021 475
DE-A-2 755 770
FR-A-2 374 283

(73) Proprietor: SHELL INTERNATIONALE RESEARCH MAATSCHAPPIJ B.V.
Carel van Bylandtlaan 30
NL-2596 HR Den Haag (NL)

(72) Inventor: Van Erp, Willibrord Adelbert
Badhuisweg 3
NL-1031 CM AMSTERDAM (NL)
Inventor: KIEFFER, Eduard Philip
Badhuisweg 3
NL-1031 CM AMSTERDAM (NL)

(74) Representative: Aalbers, Onno et al
P.O. Box 302
NL-2501 CH The Hague (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 107 875 B1

## Description

The present invention relates to a process for the preparation of an aromatic hydrocarbon mixture in which paraffins with two, three or four carbon atoms per molecule or aliphatic hydrocarbon mixtures consisting of more than 50%w of said paraffins, are contacted with a catalyst comprising a crystalline metal silicate containing one or more trivalent metals, which:

a) has been prepared by crystallisation from an aqueous mixture containing one or more compounds of an alkali metal (M), one or more organic nitrogen compounds (RN) which contain an organic cation or from which an organic cation is formed during the preparation of the silicate, one or more silicon compounds, and one or more compounds of one or more trivalent metals, and

b) after one hour's calcination in air at 500°C has an X-ray powder diffraction pattern in which the strongest lines are the four lines mentioned in Table A,

TABLE A

| d(Å) |
|---|
| 11.1 ±0.2 |
| 10.0 ±0.2 |
| 3.84±0.07 |
| 3.72±0.06 |

Such a process is known from DE—A—2755770, which describes in general terms how aromatics can be prepared from acyclic compounds having less than 6 carbon atoms, in particular ethene, propene, butene, propane and butane, or fractions containing such compounds, using as catalyst a crystalline iron silicate. The iron silicates described in this document may contain, next to iron, minor amounts of other trivalent metals such as gallium or aluminium, though this is not preferred. Applicant has continued his research in this process for the preparation of aromatics, and established that when using iron silicates prepared in accordance with the preferred embodiments of DE—A—2755770, yield and selectivity towards $C_5^+$ compounds were open to improvement. Surprisingly it has now been found that when using crystalline metal silicates in which the main trivalent metal is gallium instead of iron, in which the gallium is present in a ratio to silicium lying between certain specific limits, and in which optionally other, specific, trivalent metals are present, remarkably improved catalysts for the above process are obtained. The requirement that the main trivalent metal should be gallium is conveniently expressed by defining the molar ratio of gallium to the other, optional, trivalent metals as being above 1.

Accordingly the present invention relates to a process as defined above, characterised in that a crystalline gallium silicate is prepared from an aqueous mixture which comprises one or more gallium compounds and, if desired, one or more compounds of a trivalent metal Y chosen from the group formed by aluminium, iron, cobalt and chromium, in such quantities that in the formula representing the composition of the silicate expressed in moles of the oxides, the $SiO_2/Ga_2O_3$ molar ratio is 25—100 and the $Y_2O_3/Ga_2O_3$ molar ratio is lower than 1.

The gallium present in the catalysts occurs exclusively in the crystalline silicate and has been incorporated therein during the preparation of the silicate by crystallisation from the aqueous mixture containing one or more gallium compounds.

The preparation of the crystalline gallium silicates used in the process according to the invention may very suitably be carried out by maintaining the aqueous mixture, in which the various compounds are present in the following molar ratios expressed—with the exception of the organic nitrogen compounds—in moles of the oxides:

$M_2O:SiO_2=0.01$—$0.35$,
$RN:SiO_2=0.02$—$1.0$,
$SiO_2:Ga_2O_3=25$—$150$,
$Y_2O_3:Ga_2O_3<1$, and
$H_2O:SiO_2=5$—$65$,

at an elevated temperature until the silicate has formed, subsequently separating the silicate crystals from the mother liquor and calcining them.

In the preparation of the silicates the base mixture may very suitably be a mixture containing a quaternary ammonium compound as the organic nitrogen compound, a sodium compound as the alkali metal compound and amorphous silica as the silicon compound.

In the process according to the invention preference is given to the use of crystalline gallium silicates which have been prepared by crystallization from an aqueous mixture which, apart from possible impurities present in the reaction components, contains no compounds of a trivalent metal Y.

The silicates prepared as described hereinbefore contain alkali metal ions. By using suitable exchange methods these may be replaced by other cations, such as hydrogen ions or ammonium ions. The crystalline gallium silicates used in the process according to the invention preferably have an alkali metal

content of less than 0.05%w. In the process according to the invention the crystalline gallium silicates may be used per se or combined with a binder material, such as kaolin or bentonite.

Olefins having two, three or four carbon atoms per molecule can be converted at a relatively low temperature and in high yields into aromatic hydrocarbon mixtures by contacting the olefins with crystalline metal silicates having a similar structure as the gallium silicates used in the process according to the invention have.

A similar conversion into aromatic hydrocarbon mixtures of paraffins having two, three or four carbon atoms per molecule and of aliphatic hydrocarbon mixtures which consist more than 50%w of said paraffins is much more difficult and requires considerably higher temperatures, which accounts for the important role played by cracking reactions and the low yields of $C_5^+$ hydrocarbons. In this conversion hydrogen is released. In view of the growing demand for hydrogen for a variety of purposes it is important that in the conversion as much as possible of the hydrogen becomes available as molecular hydrogen and not in the form of hydrogen-rich by-products, such as methane. It has been found that the above-mentioned crystalline gallium silicates show a high $C_5^+$ and $H_2$ selectivity.

In the process according to the invention the starting material comprises paraffins having two, three or four carbon atoms per molecule or an aliphatic hydrocarbon mixture which consists more than 50%w of said paraffins. The paraffins with two three or four carbon atoms per molecule which should constitute more than 50%w of the feed are ethane, propane, n-butane and isobutane. If the starting material is an aliphatic hydrocarbon mixture which, in addition to the paraffins mentioned, contains other aliphatic hydrocarbons as well, this mixture may contain, inter alia, methane, ethene, propene, butene, isobutene, butadiene and paraffins and olefins with five or more carbon atoms per molecule. In the process according to the invention the starting material preferred is a feed which consists more than 75%w, and in particular substantially completely, of paraffins having three or four carbon atoms per molecule. A feedstock which is very suitable for use in the process is a mixture of paraffins with three and four carbon atoms per molecule obtained as a by-product in the production of mineral oil.

The liquid hydrocarbon mixtures obtained in the process according to the invention boil substantially in the gasoline range and have a very high octane number. They are therefore excellently suitable for use as motor gasoline or as mixing components for motor gasolines.

The process according to the invention is preferably carried out at a temperature of 350—700°C and in particular of 450—650°C, a pressure of 1—20 bar and in particular of 1—10 bar and a space velocity of 0.1—10 $kg \cdot kg^{-1} \cdot hour^{-1}$ and in particular of 0.5—5 $kg \cdot kg^{-1} \cdot hour^{-1}$.

In the process according to the invention the feed is contacted with a catalyst containing a crystalline gallium silicate which is defined, among other things, by the X-ray powder diffraction pattern which the silicate shows after one hour's calcination in air at 500°C. In this pattern the strongest lines should be the four lines mentioned in Table A. The complete X-ray powder diffraction pattern of a typical example of the present crystalline gallium silicates after one hour's calcination in air at 500°C is given in Table B.

TABLE B

| d(Å) | Rel. int. | d(Å) | Rel. int. |
|---|---|---|---|
| 11.1 | 100 | 3.84 (D) | 57 |
| 10.0 (D) | 70 | 3.72 (D) | 31 |
| 8.93 | 1 | 3.63 | 16 |
| 7.99 | 1 | 3.47 | <1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D)=doublet

In the crystalline gallium silicates used in the process according to the invention the $SiO_2/Ga_2O_3$ molar ratio is 25—100. Since investigation has shown that, within these limits, the performance of crystalline gallium silicates having a $SiO_2/Ga_2O_3$ molar ratio lower than 60 when used as catalysts for the present

conversion is indeed excellent, but that this performance is not superior to that of crystalline gallium silicates with a $SiO_2/Ga_2O_3$ molar ratio higher than 60, it is preferred in the process according to the invention, in view of the high cost of gallium, to use a catalyst containing a crystalline gallium silicate having a $SiO_2/Ga_2O_3$ molar ratio of at least 60.

The invention is now elucidated with the aid of the following example.

Example

Nine crystalline metal silicates (silicates 1—9) were prepared by heating mixtures of NaOH, amorphous silicate $(C_3H_7)_4NOH$ and $NaAlO_2$ or $Fe(NO_3)_3$ or $Ga(NO_3)_3$ in water, in an autoclave under autogenous pressure, at 150°C for 24 hours. After cooling of the reaction mixtures the silicates formed were filtered off, washed with water until the pH of the wash water was about 8 and dried at 120°C. After one hour's calcination in air at 500°C silicates 1—9 had the following properties

a) an X-ray powder diffraction pattern substantially corresponding with that mentioned in Table B, and

b) $SiO_2/Al_2O_3$, $SiO_2/Fe_2O_3$ and $SiO_2/Ga_2O_3$ molar ratios as mentioned in Table C.

TABLE C

| Silicate No. | Molar ratio $SiO_2/Al_2O_3$ | $SiO_2/Fe_2O_3$ | $SiO_2/Ga_2O_3$ |
|---|---|---|---|
| 1 | 72 | — | — |
| 2 | 260 | — | — |
| 3 | — | 72 | — |
| 4 | — | 200 | — |
| 5 | — | — | 46 |
| 6 | — | — | 70 |
| 7 | — | — | 120 |
| 8 | — | — | 180 |
| 9 | — | — | 350 |

The molar compositions of the aqueous mixtures from which silicates 1—9 were prepared may be rendered as follows:

$$w\ Na_2O \cdot 9\ (C_3H_7)_4NOH \cdot x\ Al_2O_3 \cdot y\ Fe_2O_3 \cdot z\ Ga_2O_3 \cdot 25\ SiO_2 \cdot 450\ H_2O$$

wherein w, x, y and z have the values given in Table D.

TABLE D

| Silicate No. | w | x | y | z |
|---|---|---|---|---|
| 1 | 1 | 0.33 | — | — |
| 2 | 1 | 0.06 | — | — |
| 3 | 3 | — | 0.25 | — |
| 4 | 1 | — | 0.125 | — |
| 5 | 3 | — | — | 0.5 |
| 6 | 3 | — | — | 0.33 |
| 7 | 1 | — | — | 0.2 |
| 8 | 1 | — | — | 0.125 |
| 9 | 1 | — | — | 0.06 |

From silicates 1—9 were prepared catalysts I—IX, respectively, by boiling silicates 1—9 with a 1.0 molar $NH_4NO_3$ solution, washing with water, boiling again with a 1.0 molar $NH_4NO_3$ solution and washing, drying at 120°C and calcination at 500°C.

Catalysts I—IX were tested in ten experiments (Experiments 1—10) in the preparation of $C_5^+$ aromatic hydrocarbon mixtures starting from n-butane (Experiments 1—9) and propane (Experiment 10). The experiments were carried out in a reactor containing a fixed catalyst bed. All the experiments were carried out at a temperature of 550°C, a pressure of 1.5 bar and a space velocity of 2 kg · $kg^{-1}$ · $hour^{-1}$. The results of the experiments are listed in Table E. Table F gives the composition of the $C_5^+$ products obtained in Experiments 6 and 10, carried out using catalyst VI.

TABLE E

| Feedstock | n-butane | | | | | | | | | propane | |
|---|---|---|---|---|---|---|---|---|---|---|---|
| Experiment No. | 1 | 2 | 3 | 4 | 5 | 6 | 7 | 8 | 9 | 10 | |
| Catalyst No. | I | II | III | IV | V | VI | VII | VIII | IX | VI | |
| Conversion, %w | 82 | 65 | 12 | 8 | 85 | 90 | 56 | 46 | 41 | 41 | |
| Product selectivity, %w on converted material | | | | | | | | | | | |
| $H_2$ | 1.0 | 0.6 | 3.5 | 3.1 | 5.0 | 5.0 | 3.6 | 1.8 | 2.0 | $H_2$ | 7.5 |
| $C_1$—$C_3$ | 73.8 | 79.1 | 57.3 | 55.6 | 47.2 | 45.3 | 65.0 | 69.8 | 69.0 | $C_1$—$C_2$ | 38.2 |
| iso-$C_4^\circ$ | 5.7 | 3.4 | 3.2 | 2.0 | 2.0 | 1.8 | 5.9 | 4.6 | 4.0 | $C_3^=$ | 9.2 |
| $\Sigma\ C_4^=$ | 9.0 | 7.5 | 10.2 | 29.2 | 1.4 | 1.0 | 4.9 | 7.6 | 10.2 | $\Sigma\ C_4$ | 4.0 |
| $C_5^+$ | 10.5 | 9.4 | 25.8 | 10.1 | 44.4 | 46.9 | 20.6 | 16.2 | 14.8 | $C_5^+$ | 41.1 |

TABLE F

| Feedstock | n-butane | propane |
|---|---|---|
| Experiment No. | 6 | 10 |
| Catalyst No. | VI | VI |
| Composition of $C_5^+$ product, %w | | |
| benzene | 23 | 27 |
| toluene | 41 | 37 |
| $C_8$ aromatics | 21 | 22 |
| $C_9$ aromatics | 3 | 3 |
| $C_{10}$ aromatics | 2 | 2 |
| $C_{11}^+$ aromatics | 8 | 7 |
| non-aromatics | 2 | 2 |

of the experiments mentioned in Table E only Experiments 5, 6 and 10 are experiments according to the invention. These experiments were carried out using crystalline gallium silicates having a $SiO_2/Ga_2O_3$ molar ratio in the range between 25 and 100 as catalysts. These catalysts show a high activity and a high $H_2$ and $C_5^+$ selectivity. That in Experiment 10 conversion is only 41%w as compared with 90%w in Experiment 6 is due to the fact that propane is a feedstock which is much harder to convert than n-butane. Comparison of the results of Experiments 5 (carried out using a crystalline gallium silicate having a $SiO_2/Ga_2O_3$ molar ratio in the range between 25 and 60) and 6 (carried out using a crystalline gallium silicate having a $SiO_2/Ga_2O_3$ molar ratio in the range between 60 and 100) shows that in spite of its lower gallium content this catalyst has a somewhat higher activity and $C_5^+$ selectivity than catalyst V.

Experiments 1—4 and 7—9 fall outside the scope of the invention. They have been included in the patent application for comparison.

Experiments 1 and 2 were carried out using crystalline aluminium silicates as catalysts. These catalysts show a very low $H_2$ and $C_5^+$ selectivity.

Experiments 3 and 4 were carried out using crystalline iron silicates as catalysts. These catalysts show a very low activity and a low to very low $C_5^+$ selectivity.

Experiments 7—9 were carried out using crystalline gallium silicates having a $SiO_2/Ga_2O_3$ molar ratio higher than 100. These catalysts show a relatively low activity and a low to very low $C_5^+$ selectivity.

**Claims**

1. A process for the preparation of an aromatic hydrocarbon mixture in which paraffins with two, three or four carbon atoms per molecule or aliphatic hydrocarbon mixtures consisting for more than 50%w of said paraffins, are contacted with a catalyst comprising a crystalline metal silicate containing one or more trivalent metals, which:

a) has been prepared by crystallisation from an aqueous mixture containing one or more compounds of an alkali metal (M), one or more organic nitrogen compounds (RN) which contain an organic cation or from which an organic cation is formed during the preparation of the silicate, one or more silicon compounds, and one or more compounds of one or more trivalent metals, and

b) after one hour's calcination in air at 500°C has an X-ray powder diffraction pattern in which the strongest lines are the four lines mentioned in Table A,

TABLE A

| d(Å) |
|---|
| 11.1 ±0.2 |
| 10.0 ±0.2 |
| 3.84±0.07 |
| 3.72±0.06 |

characterised in that the crystalline metal silicate is a crystalline gallium silicate prepared from an aqueous mixture which comprises one or more gallium compounds and, if desired, one or more compounds of a trivalent metal Y chosen from the group formed by aluminium, iron, cobalt and chromium, in such quantities that in the formula representing the composition of the silicate expressed in moles of the oxides, the $SiO_2/Ga_2O_3$ molar ratio is 25—100 and the $Y_2O_3/Ga_2O_3$ molar ratio is lower than 1.

2. A process as claimed in claim 1, characterized in that it is applied to a feedstock more than 75%w of which consists of paraffins having three or four carbon atoms per molecule.

3. A process as claimed in claim 2, characterized in that the feedstock consists completely of paraffins having three or four carbon atoms per molecule.

4. A process as claimed in claim 3, characterized in that the feed consists of a mixture of paraffins with three and four carbon atoms per molecule, which has been obtained as a by-product in the production of mineral oil.

5. A process as claimed in any one of claims 1—4, characterized in that it is carried out at a temperature of 350—700°C, a pressure of 1—20 bar and a space velocity of 0.1—10 $kg \cdot kg^{-1} \cdot h^{-1}$.

6. A process as claimed in any one of claims 1—5, characterized in that the crystalline gallium silicate has a $SiO_2/Ga_2O_3$ molar ratio of at least 60.

7. A process as claimed in any one of claims 1—6, characterized in that the crystalline gallium silicate has been prepared by maintaining the aqueous mixture, in which the various compounds are present in the following molar ratios, expressed—with the exception of the organic nitrogen compounds—in moles of the oxides:

$M_2O:SiO_2=0.01—0.35$,
$RN:SiO_2=0.02—1.0$,
$SiO_2:Ga_2O_3=25—150$,
$Y_2O_3:Ga_2O_3<1$, and
$H_2O:SiO_2=5—65$,

at an elevated temperature until the silicate has formed, subsequently separating the silicate crystals from the mother liquor and calcining them.

8. A process as claimed in claim 7, characterized in that the aqueous mixture contains a quaternary ammonium compound as the organic nitrogen compound, a sodium compound as the alkali metal compound and amorphous silica as the silicon compound.

9. A process as claimed in any one of claims 1—8, characterized in that the crystalline silicate has been prepared by crystallization from an aqueous mixture which, apart from possible impurities present in the reaction components, contains no compounds of a trivalent metal Y.

10. A process as claimed in any one of claims 1—9, characterized in that the crystalline gallium silicate has an alkali metal content of less than 0.05%w.

**Patentansprüche**

1. Ein Verfahren zur Herstellung einer aromatischen Kohlenwasserstoffmischung, in welcher Paraffine mit 2, 3 oder 4 Kohlenstoffatomen pro Molekül oder aliphatische Kohlenwasserstoffmischungen, die zu über 50 Gewichtsprozent aus den genannten Paraffinen bestehen, mit einem Katalysator, enthaltend ein kristallines Metallsilikat, enthaltend ein oder mehrere dreiwertige(s) Metall(e), kontaktiert werden, der

a) hergestellt wurde durch Kristallisation aus einer wäßrigen Mischung, enthaltend eine oder mehrere Verbindungen eines Alkalimetalls (M), eine oder mehrere organische Stickstoffverbindungen (RN), welche ein organisches Kation enthalten oder aus welchen ein organisches Kation während der Herstellung des Silikats gebildet wird, eine oder mehrere Siliciumverbindungen, und eine oder mehrere Verbindungen eines oder mehrerer dreiwertiger Metalle, und

b) nach einstündigem Calzinieren an Luft bei 500°C ein Röntgenpulverstreudiagramm aufweist, in welchem die stärksten Linien die 4 in Tabelle 4 dargestellten Linien sind,

TABELLE A

| d(Å) |
|---|
| 11,1 ±0,2 |
| 10,0 ±0,2 |
| 3,84±0,07 |
| 3,72±0,06 |

dadurch gekennzeichnet, daß das kristalline Metallsilikat ein kristallines Galliumsilikat ist, das aus einer wäßrigen Mischung hergestellt wurde, die eine oder mehrere Galliumverbindungen und, falls erwünscht, eine oder mehrere Verbindungen eines dreiwertigen Metals Y, ausgewählt aus der Gruppe gebildet aus Aluminium, Eisen, Kobalt und Chrom, in solchen Mengen enthält, daß in der Formel, die die Zusammensetzung des Silikats, ausgedrückt in Mol der Oxide, wiedergibt, das molare Verhältnis von $SiO_2/Ga_2O_3$ 25:1 bis 100:1 beträgt und das molare Verhältnis von $Y_2O_3/Ga_2O_3$ weniger als 1:1 beträgt.

2. Ein Verfahren, wie in Anspruch 1 beansprucht, dadurch gekennzeichnet, daß es bei einem Einsatzmaterial angewendet wird, das zu über 75 Gewichtsprozent aus Paraffinen mit 3 oder 4 Kohlenstoffatomen pro Molekül besteht.

3. Ein Verfahren wie in Anspruch 2 beansprucht, dadurch gekennzeichnet, daß das Einsatzmaterial vollständig aus Paraffinen mit 3 oder 4 Kohlenstoffatomen pro Molekül besteht.

4. Ein Verfahren wie in Anspruch 3 beansprucht, dadurch gekennzeichnet, daß das Einsatzmaterial aus einer Mischung von Paraffinen mit 3 und 4 Kohlenstoffatomen pro Molekül besteht, welche als Nebenprodukt bei der Herstellung von Mineralöl erhalten wurde.

5. Ein Verfahren wie in einen der Ansprüche 1 bis 4 beansprucht, dadurch gekennzeichnet, daß es bei einer Temperatur von 350 bis 700°C, einem Druck von 1 bis 20 bar und einer Raumgeschwindigkeit von 0,1 bis 10 $kg \cdot kg^{-1} \cdot h^{-1}$ durchgeführt wird.

6. Ein Verfahren wie in einem der Ansprüche 1 bis 5 beansprucht, dadurch gekennzeichnet, daß das kristalline Galliumsilikat ein molares Verhältnis von $SiO_2/Ga_2O_3$ von mindestens 60:1 aufweist.

7. Ein Verfahren wie in einem der Ansprüche 1 bis 6 beansprucht, dadurch gekennzeichnet, daß das kristalline Galliumsilikat durch Halten der wäßrigen Mischung, in welcher die verschiedenen Verbindungen in den folgenden Molverhältnissen vorliegen, ausgedrückt in Mol der Oxide—mit Ausnahme der organischen Stickstoffverbindungen:

$M_2O:SiO_2$=0,01:1—0,35:1,
$RN:SiO_2$=0,02:1—1,0:1,
$SiO_2:Ga_2O_3$=25:1—150:1,
$Y_2O_3:Ga_2O_3$<1:1, und
$H_2O:SiO_2$=5:1—65:1,

bei erhöhter Temperatur bis zur Bildung des Silikats, anschließendes Abtrennen der Silikatkristalle aus der Mutterlauge und Calzinieren derselben hergestellt wurde.

8. Ein Verfahren wie in Anspruch 7 beansprucht, dadurch gekennzeichnet, daß die wäßrige Mischung eine quartäre Ammoniumverbindung als organische Stickstoffverbindung, eine Natriumverbindung als die Alkalimetallverbindung und amorphes Siliciumdioxid als Siliciumverbindung enthält.

9. Ein Verfahren wie in einem der Ansprüche 1 bis 8 beansprucht, dadurch gekennzeichnet, daß das kristalline Silikat durch Kristallisation aus einer wäßrigen Mischung, welche, abgesehen von möglicherweise in den Reaktionskomponenten vorhandenen Verunreinigungen, keine Verbindungen eines dreiwertigen Metalls Y enthält, hergestellt wurde.

10. Ein Verfahren wie in einem der Ansprüche 1 bis 9 beansprucht, dadurch gekennzeichnet, daß das kristalline Galliumsilikat einen Alkalimetallgehalt von weniger als 0,05 Gewichtsprozent aufweist.

**Revendications**

1. Un procédé de préparation d'un mélange d'hydrocarbures aromatiques dans lequel des paraffines ayant deux, trois ou quatre atomes de carbone par molécule ou des mélanges d'hydrocarbures aliphatiques constitués à raison de plus de 50% en poids de ces paraffines, sont mis en contact avec un catalyseur comprenant un silicate métallique cristallin contenant un ou plusieurs métaux trivalents qui:

a) a été préparé par cristallisation à partir d'un mélange aqueux contenant un ou plusieurs composés d'un métal alcalin (M), un ou plusieurs composés organiques azotés (RN) qui contiennent un cation organique ou à partir desquels un cation organique est formé durant la préparation du silicate, un ou plusieurs composés du silicium et un ou plusieurs composés d'un ou plusieurs métaux trivalents, et

b) après calcination pendant une heure dans l'air à 500°C, a un diagramme de diffraction des rayons X par la méthode des poudres dans lequel les lignes les plus intenses sont les quatre lignes mentionnées dans le tableau A:

TABLEAU A

| d(Å) |
|---|
| 11,1 ±0,2 |
| 10,0 ±0,2 |
| 3,84±0,07 |
| 3,72±0,06 |

caractérisé en ce que le silicate de gallium cristallin est un silicate de gallium cristallin préparé à partir d'un mélange aqueux qui comprend un ou plusieurs composés du gallium et, si on le désire, un ou plusieurs composés d'un métal trivalent Y choisi dans le groupe formé par l'aluminium, le fer, le cobalt et le chrome, en quantités telles que dans la formule représentant la composition des silicates exprimée en moles des oxydes, le rapport molaire $SiO_2/Ga_2O_3$ soit de 25—100 et le rapport molaire $Y_2O_3/Ga_2O_3$ soit inférieur à 1.

2. Un procédé selon la revendication 1, caractérisé en ce qu'il est appliqué à un charge de départ qui est constituée à raison de plus de 75% en poids de paraffines ayant trois ou quatre atomes de carbone par molécule.

3. Un procédé selon la revendication 2, caractérisé en ce que la charge de départ est constituée complètement de paraffins ayant trois au quatre atomes de carbone par molécule.

4. Un procédé selon la revendication 3, caractérisé en ce que la charge consiste en un mélange de paraffines ayant trois ou quatre atomes de carbone par molécule, qui a été obtenu comme sous-produit dans la production d'huile minérale.

5. Un procédé selon l'une quelconque des revendications 1—4, caractérisé en ce qu'il est mis en oeuvre à une température de 350—700°C, une pression de 1—20 bars et une vitesse spatiale de 0,1—10 $kg \cdot kg^{-1} \cdot h^{-1}$.

6. Un procédé selon l'une quelconque des revendications 1—5, caractérisé en ce que le silicate de gallium cristallin a un rapport molaire $SiO_2/Ga_2O_3$ d'au moins 60.

7. Un procédé selon l'une quelconque des revendications 1—6, caractérisé en ce que le silicate de gallium cristallin a été préparé en maintenant le mélange aqueux, dans lequel les divers composés sont présents dans les rapports molaires suivants, exprimés, sauf en ce qui concerne les composés organiques azotés, en moles des oxydes:

$M_2O:SiO_2=0{,}01—0{,}35$
$RN:SiO_2=0{,}02—1{,}0$
$SiO_2:Ga_2O_3=25—150$
$Y_2O_3:Ga_2O_3<1$ et
$H_2O:SiO_2=5—65$

à une température élevée jusqu'à ce que le silicate soit formé, en séparant ensuite les cristaux de silicate de la liqueur-mère et en les calcinant.

8. Un procédé selon la revendication 7, caractérisé en ce que le mélange aqueux contient un composé d'ammonium quaternaire comme composé organique azoté, un composé du sodium comme composé de métal alcalin et de la silicate amorphe comme composé du silicium.

9. Un procédé selon l'une quelconque des revendications 1—8, caractérisé en ce que le silicate cristallin a été préparé par cristallisation à partir d'un mélange aqueux qui, en dehors d'impuretés possibles présentes dans les corps en réaction, ne contient pas de composés d'un métal trivalent Y.

10. Un procédé selon l'une quelconque des revendications 1—9, caractérisé en ce que le silicate de gallium cristallin a une teneur en métal alcalin de moins de 0,05% en poids.